# EUROPEAN PATENT APPLICATION

(11) **EP 4 382 609 A1**
(43) Date of publication of application: **12.06.2024**
(21) Application number: 22849576.8
(22) Date of filing: 28.07.2022
(51) Int. Cl.: C12N 15/62, A61K 31/409, A61K 38/16, A61K 47/66, A61K 49/00, A61P 35/00, C07K 14/195, C07K 16/30, C07K 19/00, C12P 21/02

(54) **FUSION PROTEIN BETWEEN ANTIGEN-BINDING MOLECULE AND STREPTAVIDIN VARIANT**

(30) Priority: 29.07.2021 JP 2021124126
(71) Applicant: The University of Tokyo, Bunkyo-ku, Tokyo 113-8654 (JP); Savid Therapeutics Inc., Tokyo 168-0064 (JP)
(72) Inventor: TANAKA, Toshiya, Tokyo 113-8654 (JP); YAMASHITA, Takefumi, Tokyo 113-8654 (JP); KODAMA, Tatsuhiko, Tokyo 113-8654 (JP); KANAI, Motomu, Tokyo 113-8654 (JP); YAMATSUGU, Kenzo, Tokyo 113-8654 (JP); TATSUMI, Toshifumi, Tokyo 113-8654 (JP); TAKAHASHI, Kazuki, Tokyo 113-8654 (JP); SUGIYAMA, Akira, Tokyo 113-8654 (JP); TSUKAGOSHI, Masanobu, Tokyo 168-0064 (JP); CHANSLER, Michael, Tokyo 168-0064 (JP); NAKAI, Masanori, Tokyo 168-0064 (JP)
(74) Representative: Godemeyer Blum Lenze Patentanwälte Partnerschaft mbB - werkpatent
(86) International application number: PCT/JP2022/029073
(87) International publication number: WO 2023/008515

(57) **Abstract**

It is an object of the present invention to provide a fusion protein of a molecule that recognizes cancer cells or the like and a mutant streptavidin, wherein the fusion protein is for use in the treatment or diagnosis of a cancer. According to the present invention, provided is A fusion protein, wherein an antigen-binding molecule having a molecular weight of 20,000 or less is bound, via a linker sequence, to the N-terminal side and/or C-terminal side of mutant streptavidin which comprises an amino acid sequence having the following mutations (1) to (6) with respect to the amino acid sequence of a core streptavidin as shown in SEQ ID NO: 17 provided that the C-terminal amino acid sequence consisting of Pro-Ser-Ala-Ala-Ser may be partially or entirely deleted, and has a decreased immunogenicity as compared with that of a wild-type streptavidin:
(1) a mutation in which the tyrosine residue at position 10 is substituted with serine or threonine;
(2) a mutation in which the tyrosine residue at position 71 is substituted with alanine or serine;
(3) a mutation in which the arginine residue at position 72 is substituted with lysine;
(4) a mutation in which the glutamic acid residue at position 89 is substituted with aspartic acid;
(5) a mutation in which the arginine residue at position 91 is substituted with lysine; and
(6) a mutation in which the glutamic acid residue at position 104 is substituted with glutamine or asparagine.

## Description

### Technical Field

The present invention relates to a fusion protein of an antigen-binding molecule and a mutant streptavidin and the use thereof.

### Background Art

Avidin and biotin, or streptavidin and biotin have an extremely high affinity between them (Kd = 10⁻¹⁵ to 10⁻¹⁴ M). This is one of the extremely strong interactions between two biomolecules. At present, the interaction between avidin/streptavidin and biotin has been widely applied in the field of biochemistry, molecular biology, or medicine. A drug delivery method and a pretargeting method have been devised, in which high binding ability between avidin/streptavidin and biotin is combined with an antibody molecule.

Patent Document 1 describes a streptavidin mutant whose immunogenicity is reduced by introducing mutations into amino acid sequence. Patent Document 1 also describes a therapeutic or diagnostic kit which comprises the above mutant streptavidin and a diagnostic or therapeutic substance labeled with biotin or a derivative thereof.

### Prior Art Documents

### Patent Documents

Patent Document 1: International Publication WO2010/95455

### Summary of Invention

### Object to be Solved by the Invention

It is an object of the present invention to provide a fusion protein of a molecule that recognizes cancer cells or the like and a mutant streptavidin, wherein the fusion protein is used to treat or diagnose cancer. It is another obj ect of the present invention to provide a means for treating cancer or a means for diagnosing cancer, in which the above-described fusion protein is used.

### Means for Solving the Object

As a result of intensive studies directed towards achieving the above-described objects, the present inventor has selected a molecule having a molecular weight smaller than that of an antibody, as a molecule that recognizes cancer cells, and then, the present inventor has prepared a fusion protein of the aforementioned molecule and a mutant streptavidin. The present inventor has found that the proliferation of cancer cells can be suppressed by photoimmunotherapy using the above-described fusion protein and a conjugate of a biotin and a phthalocyanine dye, thereby completing the present invention.

Specifically, according to the present invention, the following inventions are provided.
<1> A fusion protein, wherein an antigen-binding molecule having a molecular weight of 20,000 or less is bound, via a linker sequence, to the N-terminal side and/or C-terminal side of mutant streptavidin which comprises an amino acid sequence having the following mutations (1) to (6) with respect to the amino acid sequence of a core streptavidin as shown in SEQ ID NO: 17 provided that the C-terminal amino acid sequence consisting of Pro-Ser-Ala-Ala-Ser may be partially or entirely deleted, and has a decreased immunogenicity as compared with that of a wild-type streptavidin:
   (1) a mutation in which the tyrosine residue at position 10 is substituted with serine or threonine;
   (2) a mutation in which the tyrosine residue at position 71 is substituted with alanine or serine;
   (3) a mutation in which the arginine residue at position 72 is substituted with lysine;
   (4) a mutation in which the glutamic acid residue at position 89 is substituted with aspartic acid;
   (5) a mutation in which the arginine residue at position 91 is substituted with lysine; and
   (6) a mutation in which the glutamic acid residue at position 104 is substituted with glutamine or asparagine.
<2> The fusion protein according to <1>, which has the antigen-binding molecule having a molecular weight of 20,000 or less, the linker sequence, and the amino acid sequence of the mutant streptavidin in this order from the N-terminal side to the C-terminal side.
<3> The fusion protein according to <1> or <2>, wherein the antigen-binding molecule is a molecule that binds to an antigen expressing in a cancer cell.
<4> The fusion protein according to any one of <1> to <3>, wherein the antigen-binding molecule is a molecule that binds to Her2.
<5> The fusion protein according to any one of <1> to <4>, wherein the antigen-binding molecule has the amino acid sequence as set forth in SEQ ID NO: 2.
<6> The fusion protein according to any one of <1> to <5>, wherein the linker sequence consists of a glycine residue(s) and a serine residue(s) and the number of the amino acid residues is 5 to 25.
<7> The fusion protein according to any one of <1> to <6>, wherein the linker sequence is an amino acid sequence represented by [(Gly)ₘ-Ser]ₙ wherein m represents an integer of 1 to 10, and n represents an integer of 1 to 5.
<8> The fusion protein according to any one of <1> to <7>, which has the amino acid sequence as set forth in SEQ ID NO: 3 or SEQ ID NO: 9.
<9> A nucleic acid encoding the fusion protein having the amino acid sequence as set forth in SEQ ID NO: 3 or SEQ ID NO: 9.
<10> A cancer therapeutic agent or a cancer diagnostic agent, comprising the fusion protein according to any one of <1> to <8>.
<11> A kit for treating or diagnosing cancer, comprising (1) the fusion protein according to any one of <1> to <8>, and (2) a conjugate of biotin and a diagnostic substance or a therapeutic substance.
<12> The kit according to <11>, wherein the diagnostic substance or the therapeutic substance is a phthalocyanine dye.
<13> A method for producing the fusion protein according to any one of <1> to <8>, comprising a step of allowing a nucleic acid encoding the fusion protein according to any one of <1> to <8> to express in a host.
<14> The method according to <13>, wherein the fusion protein is allowed to express in a bacterial inclusion body and is then recovered.

### Advantageous Effects of Invention

By using the fusion protein of the present invention consisting of an antigen-binding molecule and a mutant streptavidin, for example, the proliferation of cancer cells can be suppressed.

### Brief Description of Drawings

[Fig. 1] Fig. 1 shows the results of refolding studies for MFL2-G5Sx3-His and MFL2-G5Sx3-del5.
[Fig. 2] Fig. 2 shows the results of Biacore assay (MFL2-His vs. HER2).
[Fig. 3] Fig. 3 shows the results of the Biacore assay (MFL2-His vs. Biotin-SiPc).
[Fig. 4] Fig. 4 shows the results of the MFL2-G5Sx3-His-SiPc cytotoxicity assay.
[Fig. 5] Fig. 5 shows the results of expression/purification and performance evaluation of MFL2-G5Sx1-PSAAS, MFL2-G5Sx2-PSAAS, and MFL2-G5Sx3-PSAAS.

### Embodiment of Carrying out the Invention

Hereinafter, the present invention will be described in more detail.

### < Fusion protein of antigen-binding molecule and mutant streptavidin >

The fusion protein of the present invention is a fusion protein, wherein an antigen-binding molecule having a molecular weight of 20,000 or less is bound, via a linker sequence, to the N-terminal side and/or C-terminal side of mutant streptavidin which comprises an amino acid sequence having the following mutations (1) to (6) with respect to the amino acid sequence of a core streptavidin as shown in SEQ ID NO: 17 provided that the C-terminal amino acid sequence consisting of Pro-Ser-Ala-Ala-Ser may be partially or entirely deleted, and has a decreased immunogenicity as compared with that of a wild-type streptavidin:
(1) a mutation in which the tyrosine residue at position 10 is substituted with serine or threonine;
(2) a mutation in which the tyrosine residue at position 71 is substituted with alanine or serine;
(3) a mutation in which the arginine residue at position 72 is substituted with lysine;
(4) a mutation in which the glutamic acid residue at position 89 is substituted with aspartic acid;
(5) a mutation in which the arginine residue at position 91 is substituted with lysine; and
(6) a mutation in which the glutamic acid residue at position 104 is substituted with glutamine or asparagine.

When the antigen-binding molecule having a molecular weight of 20,000 or less is bound to both the N-terminus and C-terminus of the mutant streptavidin via a linker sequence, the antigen-binding molecules may be the same or different.

Preferably, the fusion protein has the antigen-binding molecule having a molecular weight of 20,000 or less, the linker sequence, and the amino acid sequence of the mutant streptavidin in this order from the N-terminal side to the C-terminal side.

The mutant streptavidin in the present invention is a mutant streptavidin whose immunogenicity is reduced by introducing mutations into amino acid sequence, as described in International Publication WO 2010/95455. The mutant streptavidin in the present invention is a mutant streptavidin which comprises an amino acid sequence having the following mutations (1) to (6) with respect to the amino acid sequence of a core streptavidin as shown in SEQ ID NO: 17 provided that the C-terminal amino acid sequence consisting of Pro-Ser-Ala-Ala-Ser may be partially or entirely deleted,:
(1) a mutation in which the tyrosine residue at position 10 is substituted with serine or threonine;
(2) a mutation in which the tyrosine residue at position 71 is substituted with alanine or serine;
(3) a mutation in which the arginine residue at position 72 is substituted with lysine;
(4) a mutation in which the glutamic acid residue at position 89 is substituted with aspartic acid;
(5) a mutation in which the arginine residue at position 91 is substituted with lysine; and
(6) a mutation in which the glutamic acid residue at position 104 is substituted with glutamine or asparagine.

The expression "... having decreased immunogenicity as compared with that of a wild-type streptavidin" is used in the present invention to mean that, when a mutant streptavidin is administered to a mammal such as a human, the immunogenicity of the mutant streptavidin is reduced. A decrease of the immunogenicity can be confirmed by the following method, for example. That is to say, the reactivity of the mutant streptavidin of the present invention with anti-streptavidin antiserum, which has been obtained by immunizing a crab-eating monkey with a wild-type streptavidin, is analyzed. If the reactivity of the mutant streptavidin with the aforementioned anti-streptavidin antiserum is decreased as compared with that of the wild-type streptavidin, it can be determined that the immunogenicity of the mutant streptavidin is decreased as compared with that of the wild-type streptavidin. When a decrease of the immunogenicity is determined by the above-described method, the immunogenicity of the mutant streptavidin of the present invention is decreased by preferably 80% or less, more preferably 60% or less, further preferably 20% or less, still further preferably 15% or less, still further preferably 10% or less, and particularly preferably 5% or less, as compared with the immunogenicity of the wild-type streptavidin.

As described above, the fusion protein of the present invention is a fusion protein of an antigen-binding molecule having a molecular weight of 20,000 or less and a mutant streptavidin. The fusion protein of the present invention forms a tetramer as a result of the affinity between the two amino acid sequences of the mutsnt streptavidin. When the protein having the amino acid sequence described in Example 2 is, for example, used as an antigen-binding molecule, the molecular weight of the tetramer formed by the fusion protein of the present invention is approximately 96 kDa. When the fusion protein as used in the present invention is administered to a living body to treat cancer, it is important to simultaneously achieve the following points: favorable tumor uptake; good clearance rate; and favorable tumor penetration. It is conceived that the above-described molecular weight (approximately 96 kDa) of the present invention is a molecular weight, with which the above-described three parameters can be simultaneously achieved.

It may be adequate if the molecular weight of the antigen-binding molecule is 20,000 or less. The molecular weight of the antigen-binding molecule is generally 4,000 or more and 20,000 or less, preferably 4,000 or more and 10,000 or less, and more preferably 4,000 or more and 8,000 or less.

The antigen-binding molecule of the present invention is a molecule having a concept different from an antibody. The molecular weight of an IgG antibody is generally approximately 150,000, whereas the antigen-binding molecule of the present invention is a molecule having a molecular weight much smaller than that of the IgG antibody. The antigen-binding molecule of the present invention may also be a molecule produced by imitating an antibody. For example, proteins each having a molecular weight of approximately 6,000 are produced by modifying a portion of the IgG-binding domain of Protein A (VDNKFNKEQQNAFYEILHLPNLNEEQRNAFIQSLKDDPSQSANLLAEAKKLNDAQAPK) (SEQ ID NO: 18), and the thus produced proteins are then screened, so that a desired antigen-binding molecule can be selected, as appropriate.

The antigen in the antigen-binding molecule is not particularly limited. The antigen is preferably an antigen that is expressed in cancer cells. Examples of the antigen that is specifically expressed in cancer may include the following antigens:

Epiregulin (EREG), ROBO 1, 2, 3 and 4, 1-40-β-amyloid, 4-1BB, 5AC, 5T4, ACVR2B, adenocarcinoma antigen, α-fetoprotein, angiopoetin 2, anthrax toxin, AOC3 (VAP-1), B-lymphoma cells, B7-H3, BAFF, β amyloid, C242 antigen, C5, CA-125, carbonic anhydrase 9 (CA-IX), cardiac myosin, CCL11 (eotaxin-1), CCR4, CCR5, CD11, CD18, CD125, CD140a, CD147 (basigin), CD147 (basigin), CD15, CD152, CD154 (CD40L), CD154, CD19, CD2, CD20, CD200, CD22, CD221, CD23 (IgE receptor), CD25 (IL-2 receptor α chain), CD28, CD3, CD30 (TNFRSF8), CD33, CD37, CD38 (cyclic ADP ribose hydrolase), CD4, CD40, CD41 (integrin α-IIb), CD44 v6, CD5, CD51, CD52, CD56, CD6, CD70, CD74, CD79B, CD80, CEA, CFD, ch4D5, CLDN18.2, *Clostridium difficile,* clumping factor A, CSF2, CTLA-4, cytomegalovirus, cytomegalovirus glycoprotein B, DLL4, DR5, *E. coli* Shiga toxin type 1, *E. coli* Shiga toxin type 2, EGFL7, EGFR, endotoxin, EpCAM, episialin, ERBB3, *Escherichia coli,* F protein of respiratory syncytial virus, FAP, fibrin II β chain, fibronectin extra domain-B, folate receptor 1, Frizzled receptor, GD2, GD3 ganglioside, GMCSF receptor α chain, GPNMB, hepatitis B surface antigen, hepatitis B virus, HER1, HER2/neu, HER3, HGF, HIV-1, HLA-DRβ, HNGF, Hsp90, human β amyloid, human scatter factor receptor kinase, human TNF, ICAM-1 (CD54), IFN-α, IFN-γ, IgE, IgEFc region, IGF-1 receptor, IGF-I, IgG4, IGHE, IL-1β, IL-12, IL-13, IL-17, IL-17A, IL-22, IL-23, IL-4, IL-5, IL-6, IL-6 receptor, IL-9, ILGF2, influenza A hemagglutinin, insulin-like growth factor I receptor, integrin a4, integrin α4β7, integrin α5β1, integrin α7β7, integrin αIIbβ3, integrin αvβ3, integrin γ induced protein, interferon receptor, interferon α/β receptor, ITGA2, ITGB2 (CD 18), KIR2D, L-selectin (CD62L), Lewis-Y antigen, LFA-1 (CD11a), lipoteichoic acid, LOXL2, LTA, MCP-1, mesothelin, MS4A1, MUC1, mucin CanAg, myostatin, N-glycolylneuraminic acid, NARP-1, NCA-90 (granulocyte antigen), NGF, NOGO-A, NRP1, *Oryctolagus cuniculus,* OX-40, oxLDL, PCSK9, PD-1, PDCD1, PDGF-R α, phosphatidylserine, prostate cancer cells, *Pseudomonas aeruginosa*, Rabies virus glycoprotein, RANKL, respiratory syncytial virus, RHD, Rh (Rhesus) factor, RON, RTN4, sclerostin, SDC1, selectin P, SLAMF7, SOST, sphingosine-1-phosphate, TAG-72, TEM1, tenascin C, TFPI, TGFβ1, TGFβ2, TGF-β, TNF-a, TRAIL-R1, TRAII,-R2, tumor antigen CTAA16.88, MUC1 tumor-specific glycosylation, TWEAK receptor, TYRP1 (glycoprotein 75), VEGF-A, VEGFR-1, VEGFR2, vimentin, and VWF.

Among the above-described antigens, HER2 is particularly preferable.

An example of the antigen-binding molecule may be a protein having the amino acid sequence as set forth in SEQ ID NO: 2, which binds to HER2.

The linker sequence is not particularly limited, as long as the effects of the present invention can be achieved. The number of amino acids in the linker sequence is preferably 5 to 25 amino acids, more preferably 10 to 25 amino acids, and further preferably 15 to 20 amino acids.

A specific example of the linker sequence may be a sequence consisting of glycine residues and serine residues. As such a linker sequence, for example, an amino acid sequence represented by [(Gly)ₘ-Ser]ₙ (wherein m represents an integer of 1 to 10, and n represents an integer of 1 to 5) can be used. A specific example of the linker sequence may be Gly-Gly-Gly-Gly-Gly-Ser-Gly-Gly-Gly-Gly-Gly-Ser-Gly-Gly-Gly-Gly-Gly-Ser, but is not particularly limited thereto.

A specific example of the fusion protein of the present invention may be a fusion protein having the amino acid sequence as set forth in SEQ ID NO: 3 or SEQ ID NO:9.

According to the present invention, a nucleic acid (for example, DNA) that encodes the above-described fusion protein of the present invention is further provided. A specific example of the nucleic acid of the present invention may be a nucleic acid that encodes the fusion protein having the amino acid sequence as set forth in SEQ ID NO: 3 or SEQ ID NO:9. One example of the nucleic acid of the present invention may be a nucleic acid having the nucleotide sequence as set forth in SEQ ID NO: 4 or SEQ ID NO: 10.

A nucleic acid (for example, DNA) encoding the fusion protein of the present invention can be used by being incorporated into a vector. In order to produce the fusion protein of the present invention, a nucleic acid encoding the fusion protein of the present invention is incorporated into an expression vector, and a host is then transformed with this expression vector, so that the fusion protein of the present invention can be expressed in the host. According to the present invention, there is a method for producing the fusion protein of the present invention, comprising a step of allowing a nucleic acid encoding the fusion protein of the present invention to express in a host. Preferably, the fusion protein can be expressed in a bacterial inclusion body and recovered therefrom.

When *Escherichia coli* is used as a host, the vector preferably has a replication origin (ori) and also has a gene for selecting the transformed host (e.g. a drug-resistance gene that is resistant to drugs, such as ampicillin, tetracycline, kanamycin or chloramphenicol, etc.). Moreover, an expression vector preferably has a promoter capable of efficiently expressing the mutant streptavidin of the present invention in a host, such as a lacZ promoter or a T7 promoter. Examples of such a vector include an M13 vector, a pUC vector, pBR322, pBluescript, pCR-Script, pGEX-SX-1 (Pharmacia), "QIAexpress system" (QIAGEN), pEGFP, and pET (in this case, BL21 that expresses T7 RNA polymerase is preferably used as a host).

A vector can be introduced into a host cell by applying a calcium chloride method or an electroporation method, for example. Further, a sequence that encodes a tag for improving solubility, such as glutathione S-transferase, thioredoxin or a maltose-binding protein, may be added. Still further, a sequence that encodes a tag designed for facilitating purification, such as a polyhistidine tag, a Myc epitope, a hemagglutinin (HA) epitope, a T7 epitope, an Xpress tag, a FLAG tag or other known tag sequences, may also be added.

Other than *Escherichia coli,* examples of the expression vector include: mammal-derived expression vectors (for example, pcDNA3 (manufactured by Invitrogen), pEGF-BOS (Nucleic Acids. Res. 1990, 18(17), p. 5322), pEF and pCDM8); insect cell-derived expression vectors (for example, "Bac-to-BAC baculovirus expression system" (manufactured by Gibco-BRL) and pBacPAK8); plant-derived expression vectors (for example, pMH1 and pMH2); animal virus-derived expression vectors (for example, pHSV, pMV and pAdexLcw); retrovirus-derived expression vectors (for example, pZIPneo); yeast-derived expression vectors (for example, "Pichia Expression Kit" (manufactured by Invitrogen), pNV11 and SP-Q01); and *Bacillus subtilis-*derived expression vectors (for example, pPL608 and pKTH50).

When the expression of the present fusion protein in an animal cell such as a CHO cell, a COS cell or an NIH3T3 cell is intended, it is essential for the expression vector to have a promoter necessary for the expression of the fusion protein in such an animal cell, such as an SV40 promoter (Mulligan et al., Nature (1979) 277, 108), an MNILV-LTR promoter, an EF1α promoter (Mizushima et al., Nucleic Acids Res. (1990) 18, 5322) or a CMV promoter. It is more preferable if the expression vector has a gene for selecting the transformation of a cell (for example, a drug-resistance gene capable of determining transformation with the use of drugs (neomycin, G418, etc.)). Examples of a vector having such properties include pMAM, pDR2, pBK-RSV, pBK-CMV, pOPRSV and pOP13.

The type of a host cell, into which the vector is introduced, is not particularly limited. Either prokaryotes or eukaryotes may be used. It is possible to use *Escherichia coli* or various types of animal cells, for example.

In the case of using a eukaryotic cell, for example, an animal cell, a plant cell or a fungal cell can be used as a host. Examples of an animal cell that can be used herein include: mammalian cells such as a CHO cell, a COS cell, a 3T3 cell, a HeLa cell or a Vero cell; and insect cells such as Sf9, Sf21 or Tn5. When the expression of a large amount of the fusion protein in an animal cell is intended, a CHO cell is particularly preferable. A vector can be introduced into a host cell by a calcium phosphate method, a DEAE-dextran method, a method using cationic ribosome DOTAP (manufactured by Boehringer Mannheim), an electroporation method, a lipofection method or the like.

As a plant cell, a cell from *Nicotiana tabacum* is known as a protein-producing system, for example. These cells may be subj ected to callus culture. Examples of a known fungal cell include: yeast cells including genus Saccharomyces such as *Saccharomyces cerevisiae*; and filamentous fungi including genus Aspergillus such as *Aspergillus niger.*

Examples of a prokaryotic cell that can be used herein include *Escherichia coli* (*E. coli*), such as JM109, DH5α or HB101. Moreover, *Bacillus subtilis* has been known.

These cells are transformed with the nucleic acid of the present invention, and the transformed cells are then cultured *in vitro,* so as to obtain the fusion protein of the present invention. The culture can be carried out in accordance with a known culture method. Examples of a culture solution of animal cells that can be used herein include DMEM, MEM, RPMI1640, and IMDM. During the culture, a serum infusion such as fetal calf serum (FCS) may be used in combination, or serum free culture may also be carried out. The pH applied during the culture is preferably approximately pH 6 to 8. The culture is generally carried out at a temperature of approximately 30°C to 40°C for approximately 15 to 200 hours. As necessary, medium replacement, ventilation and stirring are carried out. Furthermore, growth factors may also be added to promote the growth of cells.

< Cancer therapeutic agent or cancer diagnostic agent >

The fusion protein of the present invention is useful as a cancer therapeutic agent or a cancer diagnostic agent.

According to the present invention, provided is a kit for treating or diagnosing cancer, comprising (1) the fusion protein of the present invention, and (2) a conjugate of bioyin and a diagnostic substance or a therapeutic substance.

When a molecule that is bound to an antigen existing in a cancer cell is used as such an antigen-binding molecule, the fusion protein of the present invention is administered to a patient, so that a mutant streptavidin can be accumulated specifically into cancer cells. Subsequently, by administering a conjugate of biotin and a diagnostic substance or a therapeutic substance to the patient, it becomes possible to accumulate the diagnostic substance or the therapeutic substance precisely into the cancer cells.

Otherwise, a complex is prepared by binding the "fusion protein of the present invention" with the "conjugate of biotin and a diagnostic substance or a therapeutic substance," and the thus prepared complex can be administered to the patient.

### < Conjugate of biotin and diagnostic substance or therapeutic substance >

By binding a diagnostic substance or a therapeutic substance to biotin, a conjugate of the biotin and the diagnostic substance or the therapeutic substance can be prepared. Examples of the diagnostic substance or the therapeutic substance may include a fluorochrome, a chemiluminescent agent, a radioisotope, a sensitizer consisting of a metal compound or the like, a neutron-capturing agent consisting of a metal compound or the like, a phthalocyanine dye, a low-molecular-weight compound, micro- or nano-bubbles, and a protein. Preferably, a phthalocyanine dye can be used.

### < Phthalocyanine dye >

As a phthalocyanine dye, specifically, a compound represented by the following formula (1) or a salt thereof can be used.

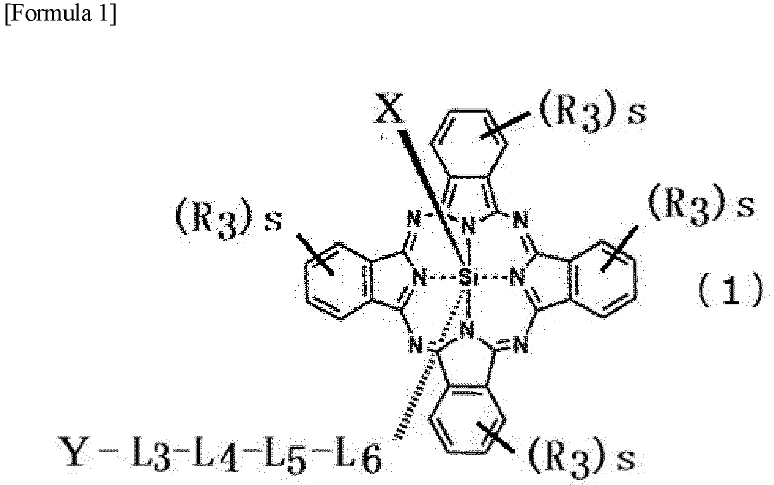

X represents a substituent having a hydrophilic group at the terminus thereof. The hydrophilic group is preferably a sulfonic acid group, a phosphoric acid group, an ammonium group, or the like.

X is preferably a substituent having a sulfonic acid group at the terminus thereof.

An example of X may be a group represented by the following formula:

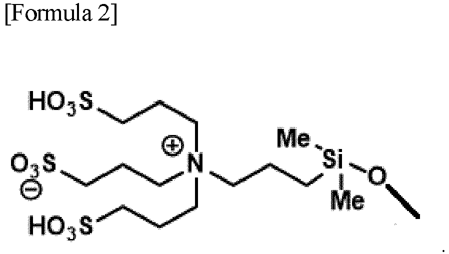

In the present description, Me represents a methyl group.

L₃, L₄, L₅, and L₆ each independently represent a divalent linking group.

L₃ is preferably the following: 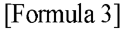 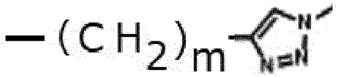 wherein m represents an integer of 1 to 5.

L₄ is preferably -[(CH₂)ₚ-O)]_{q}- (wherein p and q each independently represent an integer of 1 to 5).

L₅ is preferably -CONH-, -NHCO-, -COO-, or -OCO-, and is more preferably -CONH-.

L₆ is preferably -(CH₂)ᵣ, -(CH₂)ᵣO-, or -(CH₂)ᵣSi(R¹)(R²)-O- (wherein r represents an integer of 1 to 5, and R¹ and R² each independently represent an alkyl group containing 1 to 4 carbon atoms). R¹ and R² are particularly preferably methyl groups.

Y represents a group capable of binding to an antigen-binding molecule. Y is preferably an active ester of a carboxyl group, and is more preferably an succinimidyl ester of a carboxyl group.

R₃ represents an alkyl group containing 1 to 6 carbon atoms, an alkoxy group containing 1 to 6 carbon atoms, a halogen atom, -SR₁₁, -SOR₁₂, an aryl group containing 6 to 10 carbon atoms, - N(R₁₃)(R₁₄), or -NO₂, or two adjacent R₃s may together form an aryl group containing 6 to 10 carbon atoms. R₁₁, R₁₂, R₁₃, and R₁₄ each independently represent an alkyl group containing 1 to 6 carbon atoms or an alkoxy group containing 1 to 6 carbon atoms.

Herein, the aryl group may be optionally substituted with an alkyl group containing 1 to 6 carbon atoms or an alkoxy group containing 1 to 6 carbon atoms.

Herein, the alkyl group and the alkoxy group may be optionally substituted with a halogen atom.

When a plurality of R₃s are present, the plurality of R₃s may be identical to or different from one another.

### s represents an integer of 0 to 4. s is preferably 0.

The halogen atom may be any of fluorine, chlorine, bromine and iodine, and the halogen atom is preferably fluorine, chlorine, or bromine.

The compound of the phthalocyanine dye can be synthesized in accordance with the methods described in Production Examples 1 to 5 in the after-mentioned Examples.

### < Photoimmunotherapy >

Photoimmunotherapy is a therapeutic method of using a photosensitizer and an irradiation light to destroy specific cells in a body. When a photosensitizer is exposed to a light with a specific wavelength, it generates cytotoxic reactive oxygen species capable of inducing apoptosis, necrosis, and/or autophagy to around cells. For example, Japanese Patent No. 6127045 discloses a method of killing cells, comprising: a step of allowing cells comprising a cell surface protein to come into contact with a therapeutically effective amount of one or more antibodies-IR700 molecules, wherein the antibodies specifically bind to the cell surface protein; a step of irradiating the cells with a light at a wavelength of 660 to 740 nm and at a dose of at least 1 Jcm⁻²; and a step of allowing the cells to come into contact with one or more therapeutic agents at approximately 0 to 8 hours after the irradiation, thereby killing the cells. JP Patent Publication (Kohyo) No. 2017-524659 A discloses a method of inducing cytotoxicity to a subject affected with a disease or a pathology, comprising: (a) administering to a subject, a therapeutically effective drug comprising a phthalocyanine dye such as IRDye (registered trademark) 700DX conjugated with a probe specifically binding to the cell of the subject; and (b) irradiating the cell with an appropriate excitation light in an amount effective for inducing cell death.

The fusion protein of the present invention and the conjugate of a biotin-modified dimer and a phthalocyanine dye are administered to a subject, and the cells are then irradiated with an excitation light in an amount effective for suppression of cell proliferation or induction of cell death, so that the cell proliferation can be suppressed or the cell death can be induced, and thereby the subject can be treated.

Preferably, the fusion protein of the present invention and the conjugate of biotin and a phthalocyanine dye are administered to a subject, and the cells are then irradiated with an excitation light in an amount effective for suppression of cell proliferation or induction of cell death, so that the cell proliferation can be suppressed or the cell death can be induced, and thereby the subject can be treated.

The subj ect used herein includes humans and non-human animals. Examples of the subject may include humans and experimental animals such as mice. The subject is preferably affected with a disease regarding which suppression of cell proliferation or induction of cell death is desired. For example, the subject is affected with a cancer or a solid tumor.

Examples of the "cancer" may include carcinoma, lymphoma, blastoma, sarcoma, and leukemia or malignant lymphoma. Specific examples of the cancer may include squamous cell carcinoma (e.g., epithelial squamous cell carcinoma), lung cancer including small cell lung cancer, non-small cell lung cancer ("NSCLC"), pulmonary adenocarcinoma and pulmonary squamous cell carcinoma, peritoneal cancer, hepatocarcinoma, corpus ventriculi or stomach cancer, including digestive cancer, pancreatic cancer, glioblastoma, cervical cancer, ovarian cancer, liver cancer, bladder cancer, hepatocellular cancer, breast cancer, colon cancer, rectal cancer, colorectal cancer, endometrial membrane cancer or endometrial carcinoma, salivary gland carcinoma, kidney or renal region cancer, prostate cancer, vulvar cancer, thyroid cancer, hepatocellular carcinoma, anal carcinoma, penile carcinoma, and head and neck cancer.

The solid tumor means a benign or malignant, abnormal cell mass that generally does not contain a capsule. Examples of the solid tumor may include glioma, astrocytoma, medulloblastoma, craniopharyngioma, ependymoma, pineal gland tumor, hemangioblastoma, acoustic neuroma, oligodendrocyte, meningioma, melanoma, neuroblastoma, and retinoblastoma.

Examples of the administration method to the subject may include, but are not limited to, a local route, an injection (a subcutaneous injection, an intramuscular injection, an intradermal injection, an intraperitoneal injection, an intratumoral injection, an intravenous injection, etc.), an oral route, an ocular route, a sublingual route, a rectal route, a percutaneous route, an intranasal route, a vaginal route, and an inhalation route.

It is preferable that the conjugate of biotin and a phthalocyanine dye and the fusion protein of the present invention are each administered in a therapeutically effective amount. Regarding each of the above-described conjugate and fusion protein, the therapeutically effective amount per 60 kg is at least 0.5 mg (mg/60 kg), at least 5 mg/60 kg, at least 10 mg/60 kg, at least 20 mg/60 kg, at least 30 mg/60 kg, or at least 50 mg/60 kg. For example, when it is intravenously administered, the applied dose is 1 mg/60 kg, 2 mg/60 kg, 5 mg/60 kg, 20 mg/60 kg, or 50 mg/60 kg, and it is, for example, 0.5 to 50 mg/60 kg. In another example, the therapeutically effective amount is at least 100 µg/kg, at least 500 µg/kg or at least 500 µg/kg, and it is, for example, at least 10 µg/kg. For example, when it is intratumorally or intraperitoneally administered, the dose is 100 µg/kg, 250 µg/kg, approximately 500 µg/kg, 750 µg/kg, or 1000 µg/kg, and it is, for example, 10 µg/kg to 1000 µg/kg. In one example, when it is administered in the form of a solution for local administration, the therapeutically effective amount is 10 µg/ml, 20 µg/ml, 30 µg/ml, 40 µg/ml, 50 µg/ml, 60 µg/ml, 70 µg/ml, 80 µg/ml, 90 µg/ml, 100 µg/ml or the like, or it is 20 µg/ml to 100 µg/ml, or it is at least 500 µg/ml, or at least 1 µg/ml.

The above-described dose can be administered once or divided doses over several administrations (2, 3, or 4 times, etc.), or as a single preparation.

The conjugate of biotinr and a phthalocyanine dye and the fusion protein of the present invention can be each administered alone, or can also be administered in the presence of a pharmaceutically acceptable carrier, or can also be administered in the presence of other therapeutic agents (other anticancer agents, etc.).

The conjugate of biotin and a phthalocyanine dye and the fusion protein of the present invention can bind to target cells or target tissues, such as circulating tumor cells or solid tumor cells. Thereafter, the target cells or tissues are irradiated with a light, so that the above-described conjugate or complex can absorb the light and can damage or destroy the target cells or tissues.

In the photoimmunotherapy, the wavelength of the irradiation light is preferably 660 to 740 nm, and the irradiation light has a wavelength of, for example, 660 nm, 670 nm, 680 nm, 690 nm, 700 nm, 710 nm, 720 nm, 730 nm, or 740 nm. Light irradiation may be carried out using a device equipped with a near infrared (NIR) light emitting diode.

The light irradiation amount is at least 1 J/cm², for example, at least 4 J/cm², at least 10 J/cm², at least 15 J/cm², at least 20 J/cm², at least 50 J/cm², or at least 100 J/cm². It is, for example, 1 to 500 J/cm². Light irradiation may be carried out several times (e.g., 2, 3, 4, 5, 6, 7, 8, 9, or 10 times).

The present invention will be more specifically described in the following examples. However, these examples are not intended to limit the scope of the present invention.

### Examples

### < Production Example 1 >

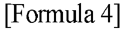

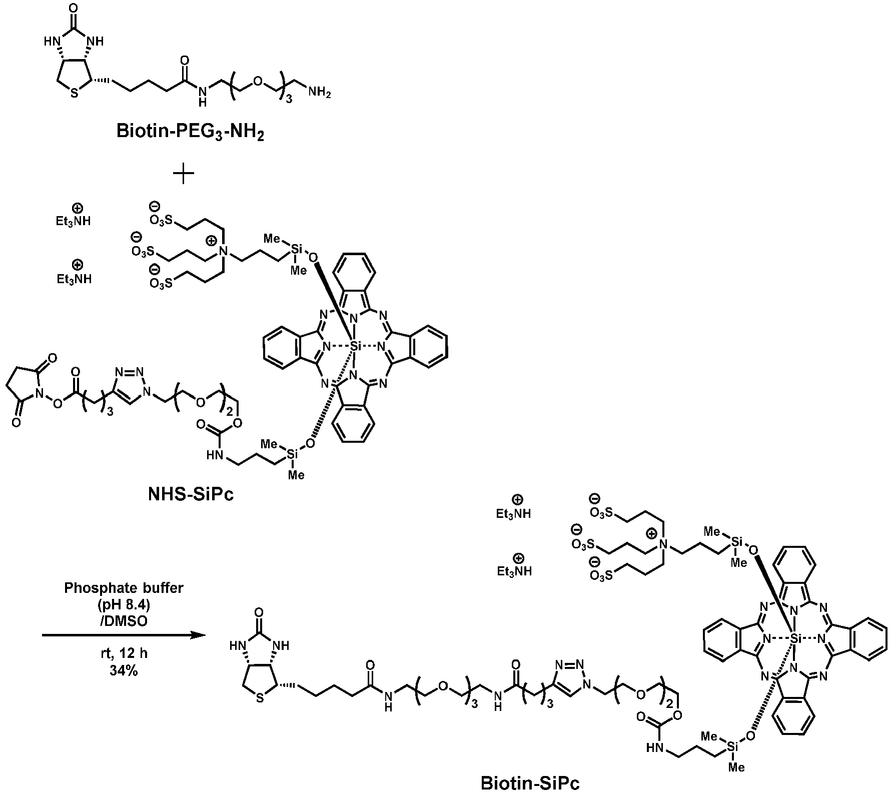

NHS-SiPc (1.8 mg, 1.0 µmol), disodium hydrogen phosphate buffer (pH 8.4, 200 µL) and dimethyl sulfoxide (200 µL) was added to biotin-PEG₃-NH2 (0.5 mg, 1.2 µmol), and the mixture was was stirred at room temperature for 12 hours while shielding from light by aluminum foil. The reaction solution was diluted to 1 mL with water and was purified by reverse phase HPLC (gradient: 30% for 5 min; 30-80% for 30 min acetonitrile in 50 mM triethylammonium acetate aqueous solution (pH 7.0), retention time = 23.8 min, YMC -Triart C18, flow rate = 3.5 mL/min) to obtain a target compound Biotin-SiPc (0.7 mg, 0.34 µmol, 34%, deep blue color).

LRMS (ESI): *m*/*z* 941.45 [M-2H]²⁻

### < Method of preparing HER2-recognizing protein >

LISA314 molecule (SEQ ID NO: 1) was fused with a molecules recognizing the HER2 antigen (Löfblom, J. FEBS Lett. 584(12): 2670-80 (2010)) (SEQ ID NO: 2), and a protein having binding ability to both biotin molecule and Her2/ErbB2 (hereafter this protein is referred to as MFL2-G5Sx3-His) was synthesized in *E*. *coli* (SEQ ID NO: 3). The gene sequence of MFL2-G5Sx3-His (SEQ ID NO: 4) was synthesized as an artificial gene by Eurofins Genomics. Using pET45b(+) as an expression vector, an expression vector was constructed according to a common method. Specifically, using a primer set (Rv: catggtatatctccttcttaaagttaaac(SEQ ID NO: 5) and Fw: cgcagcttaattaacctaggctgctgccac(SEQ ID NO: 6)), the vector was linearized by a PCR reaction. The sequence prepared by the artificial gene synthesis was amplified by a PCR reaction using a primer set(Fw: aggagatataccatgGTGGACAACAAATTCAACAAAGAG(SEQ ID NO:7), Rv: gttaattaagctgcgTTAATGATGGTGGTGATGATGCGATG(SEQ ID NO:8)). Bands were cut out of both of the PCR reaction products according to agarose gel electrophoresis, and were then purified. Using In-Fusion HD Cloning Kit (TaKaRa Bio), the purified vector and the insert were subjected to a ligation reaction and cloning in accordance with the dosage and administration described in the instruction manual.

In addition, a deletion mutant of MFL2-G5 Sx3-His expression vector was preparesd, which expresses MFL2-G5Sx3-del5 having the amino acid sequence shown in SEQ ID NO: 9, which lacks the C-terminal amino acid sequence PSAASHHHHHH of the amino acid sequence shown in SEQ ID NO: 3. Specifically, using a primer set (Fw;AAGTCAAATAACGCAGCTTAATTAACCTAG (SEQ ID NO: 11), Rw;TGCGTTATTTGACTTTGGTAAAGGTGTCATG(SEQ ID NO: 12)), a PCR reaction was carried out using the expression vector as a template. Thereafter, the reaction solution was subjected to a DpnI treatment at 37°C for 1 hour, and *E. coli* was then transformed with the obtained reaction solution, followed by cloning. After completion of the cloning, a sequence analysis was carried out to confirm that the gene sequence as set forth in SEQ ID NO: 10 was incorporated. The vector was named "pET45-MFL2-G5Sx3-del5."

Plasmid vectors (pET45-MFL2-G5Sx3-His, pET45-MFL2-G5Sx3-del5) which werre confirmed that the gene sequences shown in SEQ ID Nos 4 and 10 had been incorporated therein, was introduced into competent cells BL21(DE3) (ECOS Competent *E*. *coli* BL21(DE3), Nippon Gene Co., Ltd.), so that the cells were transformed. A culture medium prepared by culturing the cells in 100 mL of 2 × YT medium overnight was inoculated into 1 L of a culture solution, and the obtained mixture was then cultured at 37°C. When the OD value at 600 nm became 0.5 to 0.8, IPTG was added to the culture to a final concentration of 0.5 mM, and the thus obtained mixture was then cultured at 37°C for 4 hours. Thereafter, the resulting cells were recovered by centrifugation (7500 × g, 20 min at 4°C).

The method of recovering IB from the cells is described below. After Benzonase (Merck) had been added to B-PER (Thermo Scientific) and had been suspended therein, the obtained mixture was incubated at room temperature for 10 minutes, and an insoluble fraction (inclusion body; hereinafter referred to as "IB") was separated from a soluble fraction by centrifugation. Thereafter, the IB was recovered. Subsequently, the recovered IB was resuspended in a 10-fold diluted B-PER buffer (without addition of Benzonase), and was centrifuged. The supernatant was discarded, and the washing of IB was repeated three times. After completion of the washing three times, the IB recovered by centrifugation was resuspended in ultrapure water (Milli Q). Thereafter, the resuspension was dispensed in an amount of 1 mL each into 1.5-mL tubes, and was then cryopreserved at -80°C.

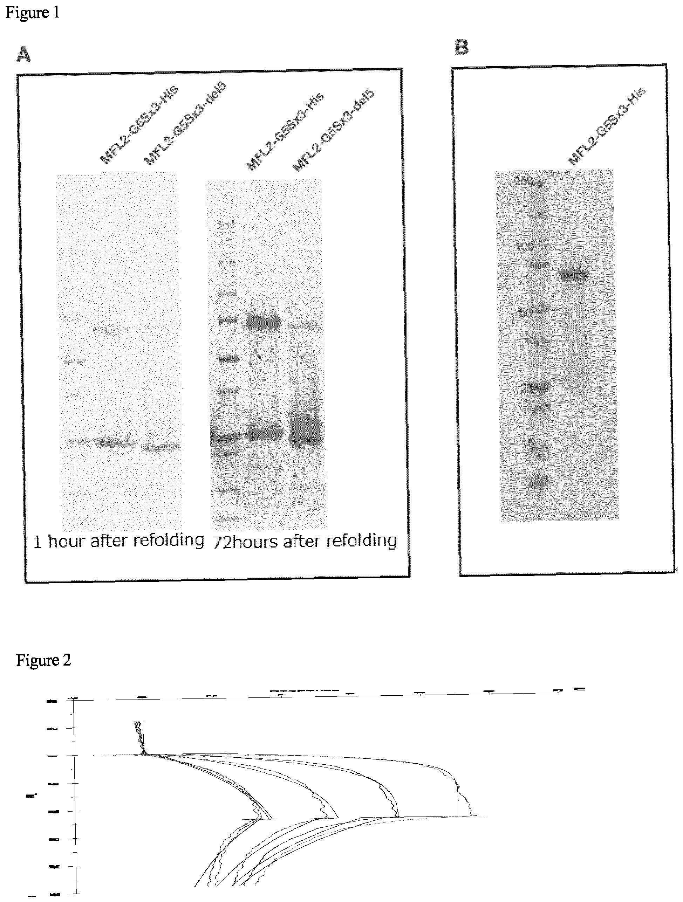

Denaturation and refolding of IB are described below. A denature buffer (0.1 M Tris-HCl, pH8.5, 6 M guanidium HCl, 10 mM EDTA) was added to IB, and the IB was then dissolved in the buffer by pipetting. Thereafter, while stirring the obtained solution using a rotator, the solution was incubated at 4°C overnight, and was then centrifuged (15,000 × *g*, 20 min at 4°C) to recover a supernatant. The recovered supernatant was adjusted with a denature buffer, so the protein concentration (OD 280 nm) in the recovered supernatant became 30 to 50 mg/mL. Thereafter, while stirring, refolding buffer (0.05 M sodium phosphate, 0.4 M arginine hydrochloride, pH6.8) was added dropwise in such an amount that the denatured protein solution having an adjusted concentration was 50-fold diluted. Thereafter, the obtained solution was incubated at 4°C for 24 to 48 hours, and was centrifuged (15,000 × *g*, 20 min at 4°C), and the supernatant was recoverd. The recovered supernatant was subjected to SDS-PAGE electrophoresis to confirm tetramer formation by CBB staining. As a result, as shown in FIG. 1A, it was confirmed that most ofMFL2-G5Sx3-del5 existed as a monomer compared to MFL2-G5Sx3-His. Regarding MFL2-G5Sx3-His, as a result of enrichment, it was confirmed that MFL2-G5 Sx3-His shown in FIG. 1B was obtained.

### < Performance evaluation of MFL2-G5Sx3-His >

MFL2-G5Sx3-His was analyzed using SPR (Biacore T200, Cytiva) in terms of the binding activity to HER2 (ErbB2). The Her2 antigen (Recombinant Human ErbB2/Her2 Fc Chimera Protein, R & D SYSTEMS) was immobilized on a Sensor Chip CM5 (Cytiva) according to an amine coupling method (Amine Coupling Kit, Cytiva). Subsequently, the binding activity was measured using the FL dilution series (2-fold dilution series from 1E-08 M to 6.25E-10 M). The results are shown in Fig. 2. From the results, it was confirmed that stable binding is shown. The analysis results were ka = 8.509E+6, kd = 0.006598, and KD = 7.753E-10.

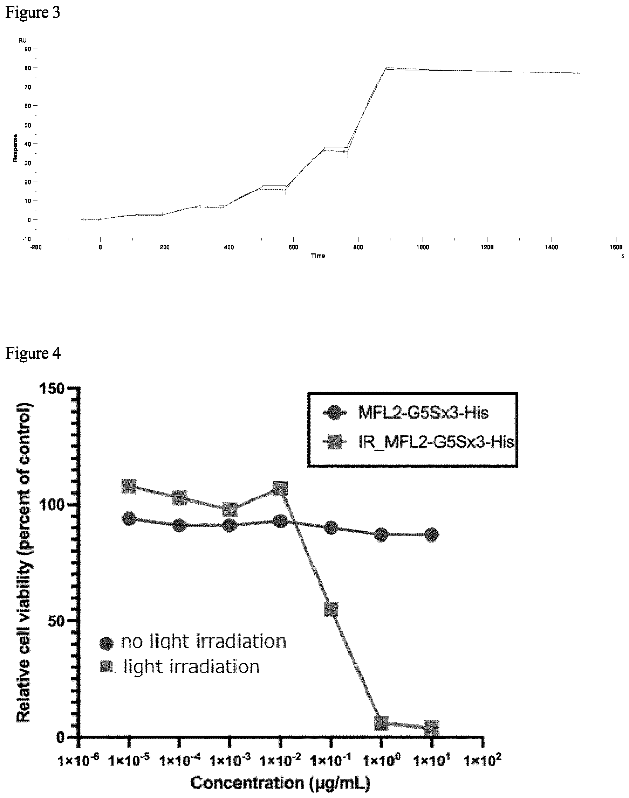

MFL2-G5Sx3-His 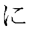 was analyzed using SPR (Biacore T200, Cytiva), in terms of the binding activity to Psyche. MFL2-G5Sx3-His was immobilized on a Sensor Chip CM5 (Cytiva) according to the amine coupling method. The binding activity was then analyzed according to a single cycle kinetics method using dilution series of biotin-SiPc (Compound Y produced in Production Example X) (twice dilution series from 1E-08 M to 6.25E-10 M: 5 seriies) as analytes. The results are shown in Fig. 3. From the results, it was confirmed that stable binding is shown. The analysis results were ka = 1.160E+6, kd = 0.002770, and KD = 2.387E-9.

### < Cytotoxicity confirmed using MFL2-G5Sx3-His and photosensitizer Psyche >.

Her2-positive cells (KPL-4) were seeded on a 96-well plate to result in a cell count of 1 × 10⁴ cells/well and a culture solution amount of 50 µL/well, and were then cultured overnight. Pirified MFL2-G5Sx3-His and biotin-SiPc (which was produced in Production Example 1) were mixed with each other to a molar ratio of 1 : 4 (hereinafter referred to as a "MFL2-G5Sx3-Hi complex"), and 8 series of 10-fold dilution series (including zero concentration) were prepared from 10 µg/mL. After the dilution series had been prepared, the complex was added to the cells, and 24 hours later, the cells were irradiated with an LED emitting a 690-nm light at 100 J/cm² from the bottom surface of the culture plate. Thereafter, the cells were cultured for 24 hours. After removing the MFL2-G5Sx3-His complex-containing medium, the cells were washed once with PBS, new medium was added, and the number of viable cells was compared using Cell Counting Kit-8 (DOJINDO LABORATORIES). A reagent was added in accordance with the dosage and administration described in the instruction manual, and the resulting cells were then incubated at 37°C in a CO₂ incubator for 1.5 hours. After that, the absorbance at 450 nm was measured, and the mean value was then calculated, followed by background correction. Thereafter, the control was set at 100%, and the cell proliferation percentage to the control under individual conditions was calculated. The results are shown in Fig. 4. It was confirmed that the complex of the FL and the photosensitizer biotin-SiPc compound has photoirradiation-dependent and concentration-dependent cytotoxicity.

### <Eexpression/purification and performance evaluation of MFL2-G4Sx1-PSAAS, MFL2-G4Sx2-PSAAS, MFL2-G4Sx3-PSAAS>

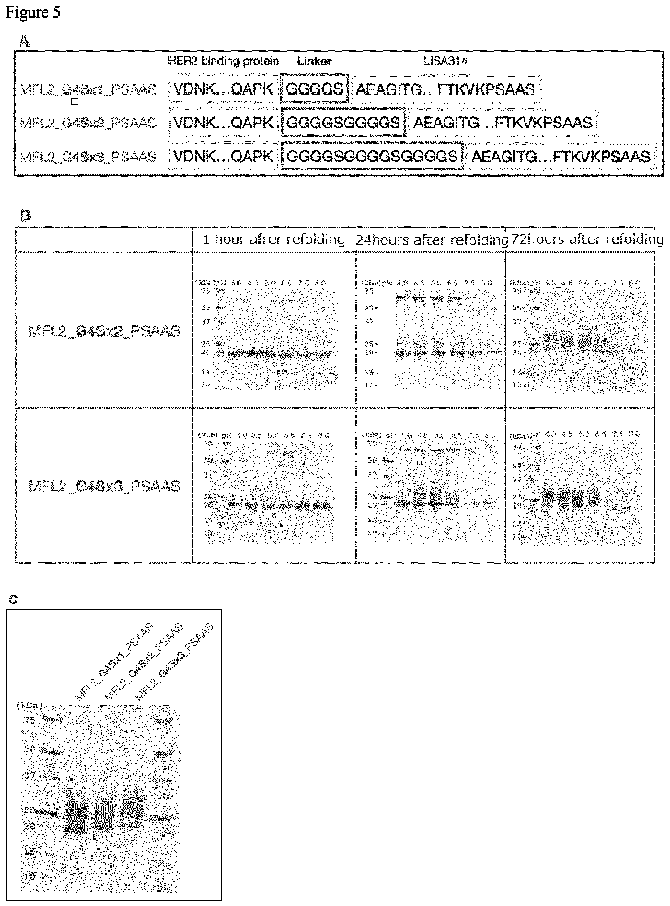

Subsequently, protein expression vectors were constructed, which has a different length of an amino acid linker that links a molecule recognizing a HER2 antigen in MFL2-G5Sx3-del5 to Cupid-del5, and has 5 ammino acids PSAAS which was added to the C-teminal. Specifically, as shown in Fig.5A, a peptide formed by combining 4 glycine residues and 1 serine residue was set to be a basic unit, and expression vectors each having a linker with a length of 1 unit, 2 units or 3 units and having PSAAS at the C-terminal were produced. The production method thereof is as follows.

Gene sequences (SEQ ID NOs: 14-16) encoding amino acid sequences having the each linker length shown in SEQ ID NOs: 11 to 13 and C-terminal were created by artificial gene synthesis. Then, using a primer set (Rv: catggtatatctccttcttaaagttaaac(SEQ ID NO:5), Fw: cgcagcttaattaacctaggctgctgccac(SEQ ID NO:6)), pET45b vector was linearized by a PCR reaction. The sequence prepared by the artificial gene synthesis was amplified by a PCR reaction using a primer set(Fw: aggagatataccatgGTGGACAACAAATTCAACAAAGAG(SEQ ID NO:7), Rv: gttaattaagctgcgTTAATGATGGTGGTGATGATGCGATG(SEQ ID NO:8)). Bands were cut out of both of the PCR reaction products according to agarose gel electrophoresis, and were then purified. Using In-Fusion HD Cloning Kit (TaKaRa Bio), the vector and the insert were subjected to a ligation reaction and cloning in accordance with the dosage and administration described in the instruction manual. Sequence analysis was carried out, and the expression vectors which were confirmed to have incorporated the gene sequences shown in SEQ ID NOs: 14-16 were named pET45-MFL2-G4Sx1-PSAAS, pET45-MFL2-G4Sx2-PSAAS, and pET45-MFL2-G4Sx3-PSAAS, respectively.

### <Recovery, denaturation and refolding purification ofIB>

pET45-MFL2-G4Sx1-PSAAS , pET45-MFL2-G4Sx2-PSAAS , pET45-MFL2-G4Sx3-PSAAS were introduced into competent cells BL21(DE3) (ECOS Competent *E. coli* BL21(DE3), Nippon Gene Co., Ltd.), so that the cells were transformed. A culture medium prepared by culturing the cells in 100 mL of 2 × YT medium overnight was inoculated into 1 L of a culture solution, and the obtained mixture was then cultured at 37°C. When the OD value at 600 nm became 0.5 to 0.8, IPTG was added to the culture to a final concentration of 0.5 mM, and the thus obtained mixture was then cultured at 37°C for 4 hours. Thereafter, the resulting cells were recovered by centrifugation (7500 × *g*, 20 min at 4°C).

The method of recovering IB from the cells is described below. After Benzonase (Merck) had been added to B-PER (Thermo Scientific) and had been suspended therein, the obtained mixture was incubated at room temperature for 10 minutes, and an insoluble fraction (inclusion body; hereinafter referred to as "IB") was separated from a soluble fraction by centrifugation. Thereafter, the IB was recovered. Subsequently, the recovered IB was resuspended in a 10-fold diluted B-PER buffer (without addition of Benzonase), and was centrifuged. The supernatant was discarded, and the washing of IB was repeated three times. After completion of the washing three times, the IB recovered by centrifugation was resuspended in ultrapure water (Milli Q). Thereafter, the resuspension was dispensed in an amount of 1 mL each into 1.5-mL tubes, and was then cryopreserved at -80°C.

Denaturation of IB is described below. A denature buffer (0.1 M Tris-HCl, pH 8.5, 6 M guanidine hydrochloride, and 10 mM EDTA) was added to IB, and the IB was then dissolved in the buffer by pipetting. Thereafter, while stirring the obtained solution using a rotator, the solution was incubated at 4°C overnight, and was then centrifuged (15,000 × g, 20 min at 4°C) to recover a supernatant. The recovered supernatant was adjusted with a denature buffer, so that the protein concentration (OD 280 nm) in the supernatant became 30 to 50 mg/mL.

Refolding is described below. The refolding buffers were prepared by adding 0.4 M arginine hydrochloride to Mcilvaine buffer and adjusting the pH value to pH4.0, pH4.5, pH5.0, pH6.5, pH7.5, pH8.0 or the like by NaOH. 25 µL of the denatured solution supernatant was added to 1 mL of refolding buffer having each pH, and rfolding was performed by dilution. The samples were incubated at 4°C, and the formation of tetramers was confirmed by SDS-PAGE electrophoresis.

For MFL2-G4Sx2-PSAAS and MFL2-G4Sx3-PSAAS, the results of confirming tetramer formation at each pH at the time of hour and 24 hours after refolding are shown in FIG. 5B. From this result, it was confirmed that tetramer formation from monomers was promoted at pH5.0-pH6.5. However, after 72 hours, it was confirmed that the tetramer band had disappeared at all pH values.

Similarly, as shown in Figure 5C, for the refolded product at pH 6.0 for MFL2-G4Sx1-PSAAS, MFL2-G4Sx2-PSAAS, and MFL2-G4Sx3-PSAAS, stable tetramer formation could not be confirmed after 72 hours.
SEQ ID NO:1 Amino acid sequence of LISA314 
AEAGITGTWSNQLGSTFIVTAGADGALTGTYESAVGNAESRYVLTGRYDSAPATDGSGTA
LGWTVAWKNNSKNAHSATTWSGQYVGGADAKINTQWLLTSGTTNANAWKSTLVGHDT
 FTKVKPSAASHHHHHH*
SEQ ID NO:2 Molecule which recognizes HER2 antigen
   VDNKFNKEMRNAYWEIALLPNLNNQQKRAFIRSIYDDPSQSANLLAEAKKLNDAQAPK
SEQ ID NO: 3 Amino acid sequence of MFL2-G5 Sx3 -Hi s 
MVDNKFNKEMRNAYWEIALLPNLNNQQKRAFIRSIYDDPSQSANLLAEAKKLNDAQAPK
SGGGGGSGGGGGSGGGGGSAEAGITGTWSNQLGSTFIVTAGADGALTGTYESAVGNAES
RYVLTGRYDSAPATDGSGTALGWTVAWKNNSKNAHSATTWSGQYVGGADAKINTQWLL
TSGTTNANAWKSTLVGHDTFTKVKPSAASHHHHHH*
SEQ ID NO:4 Gene sequence of MFL2-G4Sx3-His 
ATGGTGGACAACAAATTCAACAAAGAGATGCGCAATGCGTACTGGGAAATTGCCCTGT
TACCGAACCTGAACAACCAGCAGAAACGTGCCTTCATTCGCAGCATCTATGACGATCC
AAGCCAATCTGCGAATCTGCTTGCAGAAGCGAAGAAACTGAATGATGCTCAAGCTCC
GAAAAGCGGAGGCGGCGGGTCTGGTGGCGGTGGCAGTGGCGGAGGTGGCG
GTTCAGCGGAAGCCGGTATTACCGGGACCTGGTCCAATCAACTCGGCTCCACGTTTAT
CGTGACAGCAGGCGCGGATGGCGCGCTGACAGGGACGTACGAGTCCGCGGTAGGCA
ATGCGGAAAGCCGCTATGTGTTGACCGGTCGTTACGATTCGGCTCCGGCTACGGATGG
TTCCGGTACAGCCTTAGGGTGGACCGTTGCGTGGAAGAACAACTCGAAGAATGCCCA
CAGTGCTACCACTTGGTCAGGCCAGTATGTTGGCGGGGCGGATGCGAAAATCAACACT
CAGTGGCTTCTGACCAGCGGAACCACGAATGCCAATGCGTGGAAATCCACGCTGGTC
GGTCATGACACCTTTACCAAAGTCAAACCGAGTGCAGCATCGCATCATCACCACCATC
ATTAA
SEQ ID NO:9 Amino acid sequence of MFL2-G5Sx3-del5 
MVDNKFNKEMRNAYWEIALLPNLNNQQKRAFIRSIYDDPSQSANLLAEAKKLNDAQAPK
SGGGGGSGGGGGSGGGGGSAEAGITGTWSNQLGSTFIVTAGADGALTGTYESAVGNAES
RYVLTGRYDSAPATDGSGTALGWTVAWKNNSKNAHSATTWSGQYVGGADAKINTQWLL
 TSGTTNANAWKSTLVGHDTFTKVK
SEQ ID NO:10 Gene sequence ofMFL2-G5Sx3-del5 
ATGGTGGACAACAAATTCAACAAAGAGATGCGCAATGCGTACTGGGAAATTGCCCTGT
TACCGAACCTGAACAACCAGCAGAAACGTGCCTTCATTCGCAGCATCTATGACGATCC
AAGCCAATCTGCGAATCTGCTTGCAGAAGCGAAGAAACTGAATGATGCTCAAGCTCC
GAAAAGCGGAGGCGGCGGCGGGTCTGGTGGTGGCGGTGGCAGTGGCGGAGGTGGCG
GTTCAGCGGAAGCCGGTATTACCGGGACCTGGTCCAATCAACTCGGCTCCACGTTTAT
CGTGACAGCAGGCGCGGATGGCGCGCTGACAGGGACGTACGAGTCCGCGGTAGGCA
ATGCGGAAAGCCGCTATGTGTTGACCGGTCGTTACGATTCGGCTCCGGCTACGGATGG
TTCCGGTACAGCCTTAGGGTGGACCGTTGCGTGGAAGAACAACTCGAAGAATGCCCA
CAGTGCTACCACTTGGTCAGGCCAGTATGTTGGCGGGGCGGATGCGAAAATCAACACT
CAGTGGCTTCTGACCAGCGGAACCACGAATGCCAATGCGTGGAAATCCACGCTGGTC
GGTCATGACACCTTTACCAAAGTCAAA
SEQ ID NO: 11 Amino acid sequence of MFL2-G4Sx1-PSAA 
MVDNKFNKEMRNAYWEIALLPNLNNQQKRAFIRSIYDDPSQSANLLAEAKKLNDAQAPK
GGGGSAEAGITGTWSNQLGSTFIVTAGADGALTGTYESAVGNAESRYVLTGRYDSAPATD
GSGTALGWTVAWKNNSKNAHSATTWSGQYVGGADAKINTQWLLTSGTTNANAWKSTL
VGHDTFTKVKPSAAS*
SEQ ID NO: 12 Amino acid sequence of MFL2-G4Sx2-PSAA 
MVDNKFNKEMRNAYWEIALLPNLNNQQKRAFIRSIYDDPSQSANLLAEAKKLNDAQAPK
GGGGSGGGGSAEAGITGTWSNQLGSTFIVTAGADGALTGTYESAVGNAESRYVLTGRYDS
APATDGSGTALGWTVAWKNNSKNAHSATTWSGQYVGGADAKINTQWLLTSGTTNANA
WKSTLVGHDTFTKVKPSAAS*
SEQ ID NO: 13 Amino acid sequence of MFL2-G4Sx3-PSAAS MVDNKFNKEMRNAYWEIALLPNLNNQQKRAFIRSIYDDPSQSANLLAEAKKLNDAQAPK 
GGGGSGGGGSGGGGSAEAGITGTWSNQLGSTFIVTAGADGALTGTYESAVGNAESRYVL
TGRYDSAPATDGSGTALGWTVAWKNNSKNAHSATTWSGQYVGGADAKINTQWLLTSGT
TNANAWKSTLVGHDTFTKVKPSAAS*
SEQ ID NO: 14 Gene sequence of FL2-G4Sx1-PSAAS 
ATGGTGGACAACAAATTCAACAAAGAGATGCGCAATGCGTACTGGGAAATTGCCCTGT
TACCGAACCTGAACAACCAGCAGAAACGTGCCTTCATTCGCAGCATCTATGACGATCC
AAGCCAATCTGCGAATCTGCTTGCAGAAGCGAAGAAACTGAATGATGCTCAAGCTCC
GAAAGGAGGCGGAGGGTCTGCGGAAGCCGGTATTACCGGGACCTGGTCCAATCAACT
CGGCTCCACGTTTATCGTGACAGCAGGCGCGGATGGCGCGCTGACAGGGACGTACGA
GTCCGCGGTAGGCAATGCGGAAAGCCGCTATGTGTTGACCGGTCGTTACGATTCGGCT
CCGGCTACGGATGGTTCCGGTACAGCCTTAGGGTGGACCGTTGCGTGGAAGAACAAC
TCGAAGAATGCCCACAGTGCTACCACTTGGTCAGGCCAGTATGTTGGCGGGGCGGATG
CGAAAATCAACACTCAGTGGCTTCTGACCAGCGGAACCACGAATGCCAATGCGTGGA
AATCCACGCTGGTCGGTCATGACACCTTTACCAAAGTCAAACCGAGTGCAGCATCGTA
A
SEQ ID NO: 15 Gene sequence of MFL2-G4Sx2-PSAAS 
ATGGTGGACAACAAATTCAACAAAGAGATGCGCAATGCGTACTGGGAAATTGCCCTGT
TACCGAACCTGAACAACCAGCAGAAACGTGCCTTCATTCGCAGCATCTATGACGATCC
AAGCCAATCTGCGAATCTGCTTGCAGAAGCGAAGAAACTGAATGATGCTCAAGCTCC
GAAAGGAGGCGGAGGGTCTGGAGGTGGCGGTTCAGCGGAAGCCGGTATTACCGGGA
CCTGGTCCAATCAACTCGGCTCCACGTTTATCGTGACAGCAGGCGCGGATGGCGCGCT
GACAGGGACGTACGAGTCCGCGGTAGGCAATGCGGAAAGCCGCTATGTGTTGACCGG
TCGTTACGATTCGGCTCCGGCTACGGATGGTTCCGGTACAGCCTTAGGGTGGACCGTT
GCGTGGAAGAACAACTCGAAGAATGCCCACAGTGCTACCACTTGGTCAGGCCAGTAT
GTTGGCGGGGCGGATGCGAAAATCAACACTCAGTGGCTTCTGACCAGCGGAACCACG
AATGCCAATGCGTGGAAATCCACGCTGGTCGGTCATGACACCTTTACCAAAGTCAAAC
CGAGTGCAGCATCGTAA
SEQ ID NO:16 Gene sequence of MFL2-G4Sx3-PSAAS 
ATGGTGGACAACAAATTCAACAAAGAGATGCGCAATGCGTACTGGGAAATTGCCCTGT
TACCGAACCTGAACAACCAGCAGAAACGTGCCTTCATTCGCAGCATCTATGACGATCC
AAGCCAATCTGCGAATCTGCTTGCAGAAGCGAAGAAACTGAATGATGCTCAAGCTCC
GAAAGGAGGCGGAGGGTCTGGAGGTGGCGGTTCAGGTGGCGGTGGCAGTGCGGAAG
CCGGTATTACCGGGACCTGGTCCAATCAACTCGGCTCCACGTTTATCGTGACAGCAGG
CGCGGATGGCGCGCTGACAGGGACGTACGAGTCCGCGGTAGGCAATGCGGAAAGCCG
CTATGTGTTGACCGGTCGTTACGATTCGGCTCCGGCTACGGATGGTTCCGGTACAGCCT
TAGGGTGGACCGTTGCGTGGAAGAACAACTCGAAGAATGCCCACAGTGCTACCACTT
GGTCAGGCCAGTATGTTGGCGGGGCGGATGCGAAAATCAACACTCAGTGGCTTCTGA
CCAGCGGAACCACGAATGCCAATGCGTGGAAATCCACGCTGGTCGGTCATGACACCTT
TACCAAAGTCAAACCGAGTGCAGCATCGTAA
SEQ ID NO: 17

Ala Glu Ala Gly Ile Thr Gly Thr Trp Tyr Asn Gln Leu Gly Ser Thr
Phe Ile Val Thr Ala Gly Ala Asp Gly Ala Leu Thr Gly Thr Tyr Glu
Ser Ala Val Gly Asn Ala Glu Ser Arg Tyr Val Leu Thr Gly Arg Tyr
Asp Ser Ala Pro Ala Thr Asp Gly Ser Gly Thr Ala Leu Gly Trp Thr
Val Ala Trp Lys Asn Asn Tyr Arg Asn Ala His Ser Ala Thr Thr Trp
Ser Gly Gln Tyr Val Gly Gly Ala Glu Ala Arg Ile Asn Thr Gln Trp
Leu Leu Thr Ser Gly Thr Thr Glu Ala Asn Ala Trp Lys Ser Thr Leu
Val Gly His Asp Thr Phe Thr Lys Val Lys Pro Ser Ala Ala Ser

## Claims

1. A fusion protein, wherein an antigen-binding molecule having a molecular weight of 20,000 or less is bound, via a linker sequence, to the N-terminal side and/or C-terminal side of mutant streptavidin which comprises an amino acid sequence having the following mutations (1) to (6) with respect to the amino acid sequence of a core streptavidin as shown in SEQ ID NO: 17 provided that the C-terminal amino acid sequence consisting of Pro-Ser-Ala-Ala-Ser may be partially or entirely deleted, and has a decreased immunogenicity as compared with that of a wild-type streptavidin:
(1) a mutation in which the tyrosine residue at position 10 is substituted with serine or threonine;
(2) a mutation in which the tyrosine residue at position 71 is substituted with alanine or serine;
(3) a mutation in which the arginine residue at position 72 is substituted with lysine;
(4) a mutation in which the glutamic acid residue at position 89 is substituted with aspartic acid;
(5) a mutation in which the arginine residue at position 91 is substituted with lysine; and
(6) a mutation in which the glutamic acid residue at position 104 is substituted with glutamine or asparagine.

2. The fusion protein according to claim 1, which has the antigen-binding molecule having a molecular weight of 20,000 or less, the linker sequence, and the amino acid sequence of the mutant streptavidin in this order from the N-terminal side to the C-terminal side.

3. The fusion protein according to claim 1 or 2, wherein the antigen-binding molecule is a molecule that binds to an antigen expressing in a cancer cell.

4. The fusion protein according to any one of claims 1 to 3, wherein the antigen-binding molecule is a molecule that binds to Her2.

5. The fusion protein according to any one of claims 1 to 4, wherein the antigen-binding molecule has the amino acid sequence as set forth in SEQ ID NO: 2.

6. The fusion protein according to any one of claims 1 to 5, wherein the linker sequence consists of a glycine residue(s) and a serine residue(s) and the number of the amino acid residues is 5 to 25.

7. The fusion protein according to any one of claims 1 to 6, wherein the linker sequence is an amino acid sequence represented by [(Gly)ₘ-Ser]ₙ wherein m represents an integer of 1 to 10, and n represents an integer of 1 to 5.

8. The fusion protein according to any one of claims 1 to 7, which has the amino acid sequence as set forth in SEQ ID NO: 3 or SEQ ID NO: 9.

9. A nucleic acid encoding the fusion protein having the amino acid sequence as set forth in SEQ ID NO: 3 or SEQ ID NO: 9.

10. A cancer therapeutic agent or a cancer diagnostic agent, comprising the fusion protein according to any one of claims 1 to 8.

11. A kit for treating or diagnosing cancer, comprising (1) the fusion protein according to any one of claims 1 to 8, and (2) a conjugate of biotin and a diagnostic substance or a therapeutic substance.

12. The kit according to claim 11, wherein the diagnostic substance or the therapeutic substance is a phthalocyanine dye.

13. A method for producing the fusion protein according to any one of claims 1 to 8, comprising a step of allowing a nucleic acid encoding the fusion protein according to any one of claims 1 to 8 to express in a host.

14. The method according to claim 13, wherein the fusion protein is allowed to express in a bacterial inclusion body and is then recovered.
